# EUROPEAN PATENT APPLICATION

(11) **EP 1 500 699 A1**
(43) Date of publication of application: **26.01.2005**
(21) Application number: 03291813.8
(22) Date of filing: 22.07.2003
(51) Int. Cl.: C12N 7/00, C12N 5/06

(54) **Production of vaccinia virus with adherent or non adherent avian cell lines**

(71) Applicant: Vivalis, 49450 Roussay (FR)
(72) Inventor: Pain, Bertrand, 69003 Lyon (FR); Guehenneux, Fabienne, 44700 Orvault (FR)
(74) Representative: Warcoin, Jacques

(57) **Abstract**

The present invention relates to a method for replicating orthopoxvirus such as vaccinia virus comprising the steps of inoculating avian stem cells with viral particles and culturing said cells in a basal medium until cells lysis occurs and newly produced viral particles are released in said medium.

## Description

The present invention relates to a method for producing live or attenuated vaccinia viruses, native or modified, with avian cell lines, in particular avian stem cells, comprising infecting said cells with virus particles

Historically, vaccinia virus is known to have been used successfully to immunize against smallpox allowing its eradication in 1980 according to the WHO. Since then, vaccination has been discontinued.

Today, resurgence of this virus is considered as a potential threat that could be devastating for the unprotected population. The problem is that only 15 million doses of smallpox vaccine are available in the USA and the FDA has issued guidelines and contracts to produce large amounts of vaccine unit dose against smallpox. Further information are accessible at :
http://www.bt.cdc.gov/Agent/Smallpox/SmallpoxConsensus.pdf.

However, speeding-up production requires new production methods and suitable cell lines. In this invention, we describe new cell lines (adherent or non adherent) from avian species that could be used as substrate for vaccine production by pharmaceutical companies. These new cells are derived from avian embryos and could supersede eggs that are used at present.

Traditionally, vaccines induce immunity to diseases by using a weakened or inactivated version of the infectious agent. Today attenuated pox viruses properties like the absence of replication in human cells and the good induction of immune response allow the development of new vaccine strategies using for example MVA as vector. In fact, by classical techniques of molecular biology, it is possible to obtain recombinant MVA containing foreign DNA coding for specific peptides or proteins of therapeutic interest. These recombinant viruses after injection, in vivo, are able to stimulate the immune system against specific antigens like tumoral antigens. At present, these new generations of vaccine vectors are developed or could be developed to fight against human or animal infectious diseases and against a wide variety of tumor types (melanoma, prostate cancer, breast cancer, lung cancer, ovary cancer, liver cancer....).

Drexler I. et al, J Gen Virol. 1998 Feb;79 ( Pt 2):347-52 have observed that highly attenuated modified vaccinia virus Ankara (MVA) replicates in baby hamster kidney cells, a potential host for virus propagation, but not in various human transformed and primary cells. Therefore, the host range of MVA is restricted and this highly attenuated poxvirus strain do not create productive infections (Moss B, Dev Biol Stand 1994:55-63). In addition, production yields must be commensurate with economical viability of smallpox vaccine mass production.
For example, Blanchard TJ. et al, J Gen Virol. 1998 May;79 ( Pt 5):1159-67 have reported that modified vaccinia virus Ankara undergoes limited replication in human cells and lacks several immunomodulatory proteins.

Therefore, the purpose of the invention is to provide cell lines for replicating the vaccinia virus which would obviate the above mentioned problems and which would meet with regulatory agency requirements.

In this regards, we investigated the use of avian cells for replicating viruses. However, to maintain avian stem cells *in vitro* for long periods of time, it is necessary to observe specific culture and maintenance conditions as described in Pain et al., 1996; US 6,114,168 and EP 787 180 and these culture conditions are cost demanding.

In addition, because unlimited cell proliferation is required for the process of vaccine mass production, we choose to examine the avian stem cell ability for replicating viruses. But, the problem is to be able to maintain avian stem cells in culture in an economical medium while avoiding stumbling blocks such as cellular differentiation and senescence.

In the context of the invention, it has been found that the withdrawal of growth factors, serum and/or feeder layer leads to the isolation of populations of stem cells, in particular of somatic stem cells, which can grow indefinitely in basic culture media.

Also, apart from the hematopoietic stem cells which are for the most part nonadherent cells, the cells obtained according to the prior art techniques exhibit an adherent phenotype. Now, the industrial use of cells, as viral replication support, favors nonadherent cells. This phenotype is advantageous both because of ease of handling which avoids the use of a proteolytic enzyme for dissociation and for the high densities reached by nonadherent cells cultured *in vitro.*

The present invention describes the production of lines which can become spontaneously nonadherent or for which the nonadherence is obtained by a withdrawal of the feeder layer. Because of their growth in suspension, these lines are perfectly suitable for industrial use for the production of vaccines in bioreactors.

In addition to their properties of growing on a basic culture medium, it has been discovered that these cell lines allow the replication of certain viruses in yields equivalent to or even higher than the yields obtained with current methods, which makes these cells particularly useful for the mass production of vaccines.

Here, we report that the established new avian stem cell lines detailed in our copending application PCT/FR 03/00735, are particularly suitable for replicating orthopoxvirus such as the vaccinia virus.

### Description

Thus, in a first aspect, the present invention relates to a method for replicating vaccinia virus such as native or recombinant vaccinia virus comprising the steps of inoculating said avian stem cells with viral particles and culturing said cells in a basal medium until cells lysis occurs and newly produced viral particles are released in said medium. Inoculation is performed with an m.o.i. (multiplicity of infection) of 0.01 to 0.5. This method is useful for producing a vaccine against poxviridae in particular against smallpox.

Said avian stem cell lines are obtainable by a process consisting of:
a) culturing avian cells in a medium containing all the factors allowing their growth and an inactivated feeder layer,
b) passage by modifying the culture medium so as to obtain progressive or total withdrawal of said factors, of the serum and/or of the feeder layer,
c) establishing adherent or non adherent cell lines capable of proliferating in a basal medium in the absence of exogenous growth factors, serum and/or inactivated feeder layer.

This process leads to the establishment of new lines which are maintained in culture *in vitro* over a considerable period of time. Advantageously, the cells derived from the lines obtained in step c) are capable of proliferating for at least 50 days, 100 days, 150 days, 300 days or preferably at least 600 days. The 600 days do not constitute a time limit because the cell lines obtained are still alive after much longer time periods. Hence, these lines are considered as being able to grow indefinitely in a basic culture medium free of exogenous growth factors, serum and/or inactivated feeder layer. The expression "line" is understood to mean any population of cells capable of proliferating indefinitely in culture *in vitro* while retaining to a greater or lesser degree the same morphological and phenotypic characteristics.

The cells derived from the lines according to the invention may be avian stem cells, in particular avian somatic stem cells.

Of course, the method mentioned above makes it possible to obtain cellular clones derived from cells obtained from established lines. These clones are cells which are genetically identical to the cell from which they are derived by division.

In a particular embodiment, the invention relates to a method as defined above, in which the established lines are adherent stem cells which proliferate in the absence of inactivated feeder layer.

In this regard, in the method described above, step b) consists in a withdrawal of the components of the medium (growth factors alone or serum alone or growth factors and then serum or alternatively serum and then growth factors).

In another embodiment, the invention relates to a method as defined above in which the established lines are non adherent stem cells which proliferate in suspension in a medium free of exogenous growth factors.

In this regard, in the method described above, step b) consists in a progressive or total withdrawal of the feeder layer and then optionally in a withdrawal of the other components of the medium (growth factors and serum).

In another embodiment, the invention relates to a method as described above in which the established lines are non adherent stem cells which proliferate in suspension in a medium free of serum (serum-free medium).

In another embodiment, the invention relates to a method as defined above, in which the established lines are non adherent stem cells which proliferate in suspension in a medium free of exogenous growth factors and serum.

In another alternative, step b) consists in a progressive or total withdrawal of the growth factors, optionally followed by a progressive withdrawal of the serum.

In another alternative, step b) consists in a progressive or total withdrawal of the growth factors and/or serum, optionally followed by a withdrawal of the feeder layer.

In addition, the established lines may be cells which proliferate in a serum-depleted medium, in particular in a medium free of serum. The expression serum-depleted is understood to mean a gradual reduction of the concentration of serum spread out over time. This method allows a selection of clones which adapt to these new, increasingly drastic conditions until stable lines are obtained which are capable of growing in a serum-depleted medium or in a medium completely free of serum.

The method described above may additionally comprise a step in which the cells obtained in step c) are subjected to a selection or an adaptation in culture media used for large-scale production so as to obtain clones suitable for the production of vaccines intended for human or animal therapy.

The cells according to the invention have at least one of the following characteristics:
- a high nucleocytoplasmic ratio,
- an endogenous alkaline phosphatase activity,
- an endogenous telomerase activity,
- a reactivity with specific antibodies selected from the group of antibodies SSEA-1 (TEC01), SSEA-3, and EMA-1.

Preferably, the cells of the invention have all the above mentioned characteristics.

These cell lines and the cells derived therefrom are capable of proliferating for at least 50 days, 100 days, 150 days, 300 days, or preferably at least 600 days in a basal medium, in particular in a medium such as DMEM, GMEM, HamF12 or McCoy supplemented with various additives commonly used by persons skilled in the art. Among the additives, there may be mentioned nonessential amino acids, vitamins and sodium pyruvate.

These cell lines and the cells derived thereof are avian stem cells, in particular avian somatic stem cells or avian embryonic stem cells.

Advantageously, the cells derived from established lines are modified in order to produce an attenuated virus which is a modified vaccinia virus and/or recombinant vaccinia. Said cells may be modified by any technique accessible to persons skilled in the art, in particular by non homologous or homologous, directed and/or conditional recombination (Cre-Lox or FLP-FRT system), by transformation with any vector, plasmid, viruses or recombinant viruses in particular with the aid of retroviruses or recombinant retroviruses.

In one particular embodiment, the invention is directed to a method to produce live or attenuated vaccine such as a vaccine against smallpox comprising culturing the adherent or non adherent cell lines established in step c) according to the process described above, inoculating said cells with viral particles and culturing said cells in a basal medium as mentioned above until cell lysis occurs and newly produced viral particles are released in said medium. The invention has shown to be particularly useful for the production of attenuated virus belonging to the family of orthopoxvirus, in particular vaccinia virus, modified vaccinia virus such as Modified Vaccinia virus Ankara (MVA) which can be obtained from ATCC (ATCC Number VR-1508), and recombinant vaccinia virus. For example, one can use recombinant MVA to express antigen against diseases and infections, such as cancer and smallpox.

Preferably, the invention is aimed at the use of the non-adherent cells as defined above to produce live or attenuated vaccine comprising culturing the adherent or non adherent cell lines established in step c) according to the process described above, inoculating said cells with viral particles and culturing said cells in a basal medium as mentioned above until cell lysis occurs and newly produced viral particles are released in said medium. The invention has shown to be particularly useful for the production of attenuated virus belonging to the family of orthopoxvirus, in particular vaccinia virus, modified vaccinia virus such as Modified Vaccinia virus Ankara (MVA) and recombinant vaccinia virus.

For the remainder of the description, reference will be made to the legend to the figures below.

### Legend

**Figures 1-3: Growth curves for the cell lines of the invention** (with withdrawal of serum (fig. 2) and with withdrawal of feeder layer (fig. 3).
**Figures 4: Photograph showing the characteristic morphology of avian stem cells**
   N: nucleus, n: nucleolus and C: cytoplasm
   (isolate S86N99, X40 magnification, photograph taken with a Sony Cyber-shot digital camera)
**Figures 5: Photograph showing the alkaline phosphatase activity of avian stem cell lines which are adherent or which are in suspension**

After fixing (0.1% formaldehyde/0.5% glutaraldehyde, 30 minutes at 4°C), the cells are rinsed twice in 1X PBS and incubated for between 10 and 30 minutes at 37°C in an NBT/BCIP (Nitro Blue Tetrazolium chloride 0.375 mg/ml, 5-bromo-4-chloro-3-indolyl phosphate 0.188 mg/ml, 0.1M Tris pH 9.5, 0.05M MgCl₂, 0.1M Nacl) solution. The reaction is stopped by two 1X PBS washes and the photographs are taken.
**A-** illustrates the characteristic violet coloration of the endogenous alkaline phosphatase activity obtained with the adherent line S86N45 p87, a line cultured with no feeder or factor (X40 magnification, Sony Cyber-shot digital camera).
**B-** illustrates the violet coloration characteristic of the endogenous alkaline phosphatase activity obtained with the EB14 line maintained from 8 passages in suspension, line derived from the S86N45 cells, cultured in suspension with no feeder or factor (X20 magnification, Sony Cyber-shot digital camera).

### Example 1: Production and establishment of the adherent cells

The eggs are opened, the yolk is separated from the egg white during the opening. The embryos are removed from the yolk either directly or with the aid of a Pasteur pipette, or with the aid of a small absorbent filter paper (Whatmann 3M paper), cut out beforehand in the form of a perforated ring with the aid of a punch. The diameter of the perforation is about 5 mm. These small rings are sterilized using dry heat for about 30 minutes in an oven. This small paper ring is deposited on the surface of the yolk and centered on the embryo which is thus surrounded by the paper ring. The latter is then cut out with the aid of small pairs of scissors and the whole removed is placed in a Petri dish, filled with PBS or with a physiological saline. The embryo thus carried away by the ring is cleaned of the excess yolk in the medium and the embryonic disk, thus freed of the excess vitellin, is collected with a Pasteur pipette.

In both cases, the embryos are placed in a tube containing physiological medium (1X PBS, Tris Glucose, medium, and the like). The embryos are then mechanically dissociated and inoculated on a "feeder" into defined culture medium. Among the preferred conditions used for the culturing, preference is given to the culture medium composed of MacCoy medium as basal medium supplemented with fetal calf serum at an initial concentration of 12 to 8%, with nonessential amino acids at 1%, with a mixture of vitamins of commercial origin at 1%, with sodium pyruvate at a final concentration of 1 mM, with beta-mercaptoethanol at a final concentration of 0.2 mM, glutamine at a final concentration of 2.9 mM, with an initial mixture of antibiotics containing gentamycin at a final concentration of 10 ng/ml, penicillin at a final concentration of 100 U/ml and streptomycin at a final concentration of 100 µg/ml. Rapidly after the first passages of the cells, the mixture of antibiotics is no longer added to the medium. The expression rapidly is understood to mean after the first 3 to 5 passages in general. A mixture of nucleosides may also be added, this mixture being prepared as described above (Pain et al., 1996). Among the basal media tested under these same conditions and which give similar results are the HamF12, Glasgow MEM and DMEM media, the latter supplemented with biotin at a final concentration of 8 mg/l. By way of comparison, the biotin concentration is 0.2 mg/l in the MacCoy medium, 0.0073 mg/l in the HamF12 and 0 in the commercial DMEM and GMEM media.

The growth factors and the cytokines added to the culture medium are preferably factors and cytokines which are recombinant, including mouse SCF at a final concentration of 1 ng/ml, IGF-1 at a final concentration of 1 to 5 ng/ml, CNTF at a final concentration of 1 ng/ml, IL-6 at a final concentration of 1 ng/ml, and the soluble IL-6 receptor at a final concentration of 0.5 ng/ml to 1 ng/ml. In some experiments, some other factors may be added during the first passages. For example up to passage 3 or 10, it is possible to add bFGF to the medium at a final concentration of 1 ng/ml and IL-11 at a final concentration of 1 ng/ml.

The inoculation is carried out into this medium on the inactivated "feeder" composed of mouse fibroblasts established as lines, the STO cells. In some cases, these cells were transfected with simple expression vectors allowing the expression of growth factors such as avian SCF, constitutively in the STO cells. Thus, this "feeder" produces the factor in a form which is soluble and/or attached in the plasma membrane of the cells.

After initial inoculation of the cells directly into this medium, fresh medium can be added or the medium can be partially changed the next day, and then partially or completely during subsequent days, depending on the rate of adhesion observed for the primary cells. After about 4 to 7 days depending on the cases, the initial culture is dissociated and transferred into new dishes in the same initial medium on the inactivated feeder. After three to five passages, the cells are cultured on an inactivated feeder of STO cells which are nontransfected or transfected with an expression vector encoding a resistance to an antibiotic such as the gene for resistance to neomycin, to hygromycin, to puromycin and the like. After about twenty passages, the cells are progressively deprived of growth factors and cytokines. The expression gradual withdrawal is understood to mean a removal factor by factor from the culture medium. Thus, at one passage, SCF is first of all removed, and then, two or three passages later, IGF-1. If the cells do not exhibit morphological alterations or a variation in their average rate of proliferation, the other factors, such as CNTF and IL-6, are then removed. This withdrawal may also be drastic. All the factors are in this case removed all at once. The cells are then observed and are only passaged several days later if their rate of proliferation is modified. The latter solution is generally that which is practiced.

Various isolates are thus obtained and maintained for very long periods of time. The expression very long periods of time is understood to mean periods of the order of several weeks with a minimum of 50 days, preferably periods greater than 200 to 400 days, without limitation in time. Periods greater than 600 days are observed.

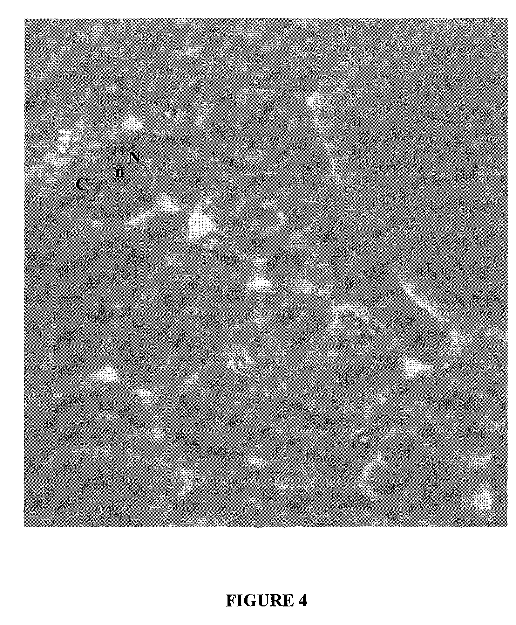

Regardless of the support used, all the cells which are adherent are dissociated with a proteolytic dissociation enzyme, such as pronase, collagenase, dispase, trypsin, and the like. Preferably, a proteolytic enzyme of bacterial origin is used in order to avoid any potential contaminant of animal origin. These cells have the characteristics of embryonic stem cells with a specific morphology illustrated, by way of example, by the **photograph of figure 4** i.e. a small size, a large nucleocytoplasmic ratio, a nucleus with at least one nucleolus which is clearly visible and a very small cytoplasm. These cells are characterized by growth in the form of more or less compact solid masses. The adherent and nonadherent cells exhibit cross-reactivity with a number of antibodies, as described above in Pain et al., 1996 and in patents US 6,114,168 and EP 787 180. The endogenous telomerase activity component is also present and is an important factor in the "stem" nature of these cells.

Cells of different isolates are obtained and maintained for long periods of time.

**Table 1**

| illustrates a few of the characteristics of these isolates | | | | | | |
|---|---|---|---|---|---|---|
| ***Name*** | **Species** | **Start** | **"Stoppage"** | **Days** | **Passage** | **Generation** |
| | | | | | | |
| S86N16 | Chicken S86N | 26-01-2000 | 05-08-2001 | 559 | 207 | 692 |
| WL3 | Chicken WL | 28-06-2000 | 09-08-2001 | 403 | 153 | 333 |
| Valo4 | Chicken Valo | 26-09-2000 | 07-02-2002 | 401 | 135 | 317 |
| S86N45 | Chicken S86N | 29-01-2001 | 12-11-2001 | 287 | 118 | 329 |

It will be noted that the term "stoppage" does not correspond to the end of the proliferation of the cells but to a deliberate stoppage of the cell cultures by the experimenter. The number of generation n is obtained by the formula X = 2ⁿ or X is the theoretical cumulative number of cells. This number is available since the cells are counted at each passage and during each inoculation. The complete history of the culture is thus available.

### Example 2: Passage of the cells

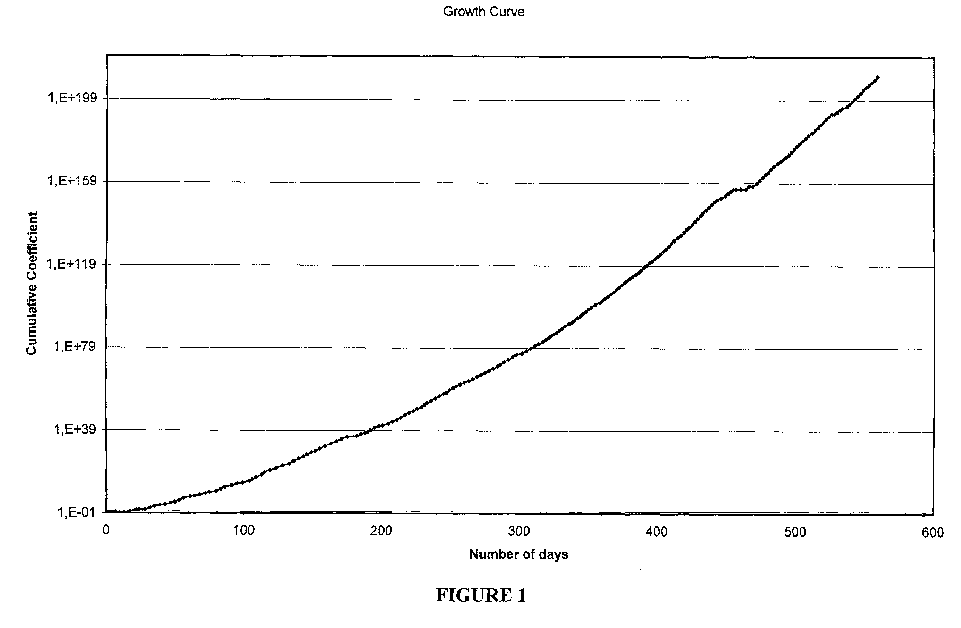

One of the characteristics of stem cells, in particular somatic stem cells and embryonic stem cells, is their capacity to proliferate *in vitro* for considerable periods of time. In order to propagate and to passage the cells, the culture medium is changed and replaced with fresh medium a few hours before their passage. The curve presented in **figure 1** illustrates a profile of cell growth and establishment.

### Example 3: Doubling time and average division time

Starting with the established cells in culture and the cells presented in the preceding examples, a mean division time can be calculated. For all the independent isolates obtained, the rate of proliferation increases slightly during successive passages, thus causing the average division time during the establishment of the cells to vary. In the adherent phase, the cells are initially inoculated on an inactivated feeder layer and are passaged regularly at a constant initial inoculation density of 1 to 2 × 10⁶ cells per 100 mm dish. Table 2 illustrates the doubling time (d) and the mean division time (MDT in hour) for 3 established cell types as a function of the culture time. It is observed that the mean doubling time decreases during the establishment.

**Table 2:**

| Cells/days | 50 | 100 | 150 | 200 | 250 | 300 | 350 | 400 | 450 | 500 | 550 |
|---|---|---|---|---|---|---|---|---|---|---|---|
| | | | | | | | | | | | |
| S86N16 (d) | 0.30 | 0.63 | 1.00 | 0.86 | 1.13 | 1.15 | 1.47 | 1.70 | 1.94 | 1.50 | 1.9 |
| S86N16 (MDT) | 80 | 38 | 24 | 27.9 | 21.2 | 20.9 | 16.3 | 14.1 | 12.4 | 16 | 12.6 |
| S86N45 (d) | 0.49 | 0.89 | 0.89 | 1.45 | 2.15 | x | x | x | x | x | x |
| S86N45 (MDT) | 49 | 26.8 | 27 | 16.5 | 11.1 | x | x | x | x | x | x |
| Valo4(d) | 0.03 | 0.61 | 1.00 | 1.17 | 1.26 | 1.03* | 1.08* | 1.25* | x | x | x |
| Valo4 (MDT) | >48 | 39.3 | 24 | 20.5 | 19 | 23.3 | 22.2 | 19.2 | x | x | x |
| The mean doubling time d is established for the period of time indicated in days with the following formula: d = (1/Log2 × (LogX2/X1))× 1/(T2-T1) where X2 and X1 are total numbers of cells at the times T2 and T1. This formula is the direct consequence of the calculation of the number of generations N by the formula X = 2ⁿ presented in example 1. The mean division time (MDT)is then obtained in hours by dividing 24 hours by d. | | | | | | | | | | | |

| | | | | | | | | | | | |
|---|---|---|---|---|---|---|---|---|---|---|---|
| * The Valo cells are passaged during this establishment on a plastic support without the presence of a feeder. The doubling time decreases and then increases again, when the cells become rehabituated to this new environment. | | | | | | | | | | | |

### Example 4: Control of the level of serum for the proliferation of the lines

During the obtaining of these lines, the culture media used are conventional culture media comprising a base (DMEM, GMEM, HamF12, McCoy, and the like) supplemented with various additives such as nonessential amino acids, vitamins, and sodium pyruvate. This complex medium comprises fetal calf serum, which remains a central component of the culture, even though components of different origins, including plant components, can be gradually used. A process for controlling and habituating the cells to relatively low proportions of fetal calf serum is presented. It is thus possible to maintain cells in high proliferation (division time >1) with low percentages of serum (2% for example in the case of the S86N16 cells).

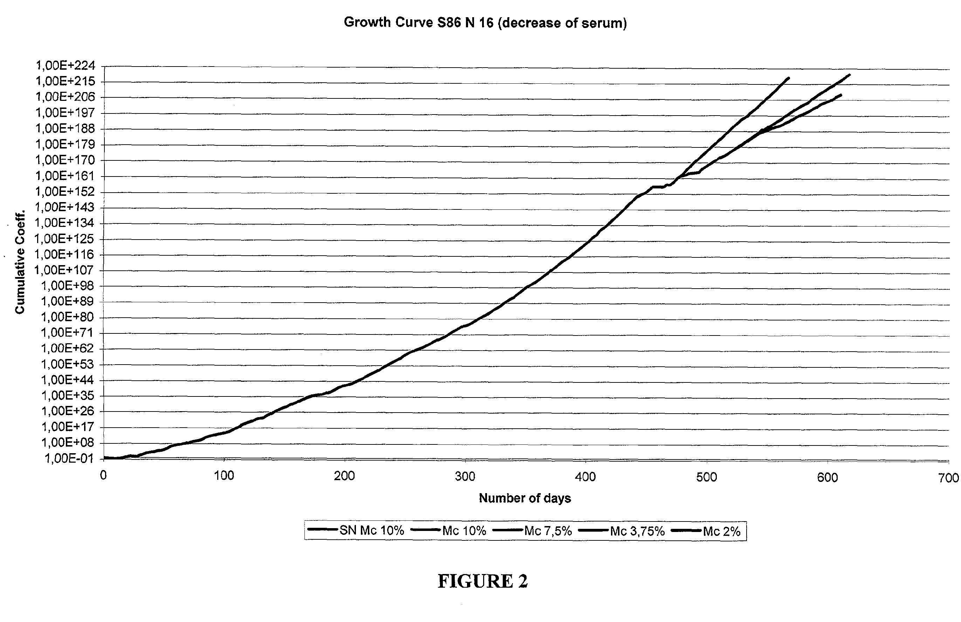

The curves presented in **figure 2** illustrates the relative reduction of serum for a given cell type: S86N16 cells, The doubling time and the mean division times were also calculated and presented in **table 3**. It will be noted that the mean division time increases as a function of the relative reduction in serum. A recovery phase is nevertheless observed after some time in culture under the conditions mentioned. This time remains nevertheless less than 24 h (d>1), which already represents a very advantageous proliferation in industrial terms even at serum concentrations of 2%, which is already relatively low. Improvements with regard to the different metabolites to be used may be envisaged in order to increase this time and still further optimize the culture conditions.

**Table 3:**

| Condition | 10% | 7.5% | 3.75% | 2% |
|---|---|---|---|---|
| d | 2.02 | 1.51 | 1.47 | 1.08 |
| MDT | 11.9 | 15.8 | 16.3 | 22.2 |

The examples are taken between passages p204 and p179 for the 10% condition, between p198 and p176 for the 7.5%, between p224 and p201 for the 3.75% and between p216 and p 199 for the 2%.

### Example 5: Deprivation of the cells of feeder layer

Under the initial culture conditions, the presence of a layer of inactivated cells appears to be necessary in order to obtain embryonic stem cells as was described above. This feeder layer no longer appears to be necessary after a number of passages. Only the "culture treated" plastic appears to be important. Indeed, one of the characteristics of some eukaryotic cells is to proliferate in adherent form. In order to facilitate the adhesion of the cells, the various plastic materials used are "culture" treated. They undergo during their manufacture a treatment which adds charges at the surface of the plastic, which charges promote the adhesions of the extracellular matrix of the cells. By contrast, the cell culture untreated plastic, often called plastic of bacteriological quality, is not surface treated by addition of specific feeders. The adhesion of the cells thereto is generally very difficult, or even impossible, or then induces changes in morphology, and in behavior which are often drastic. This distinction between the two plastic qualities makes it possible to obtain, depending on the inoculations which are carried out therein, cells with different behaviors. Gradual deprivation of the cultures of inactivated "feeder" makes it possible to obtain, after a few passages, homogeneous cultures of stem cells directly inoculated on "culture treated" plastic.

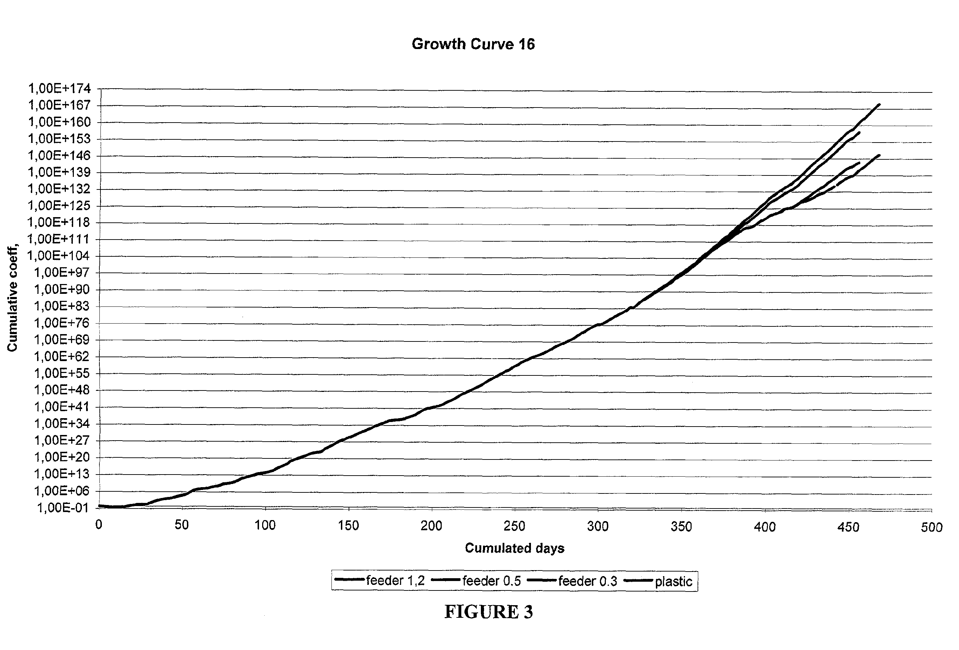

The comparative growth curves for the cells maintained in the presence and in the absence of inactivated "feeder" are presented with the case of the S86N16 cells in **figure 3**. This adaptation of the cells is progressive so as not to lose the stem cell character of the cells initially maintained on a "feeder". Progressive derivatives are thus made. The obtaining of cells which proliferate on plastic is the accomplishment of the withdrawal process. In **table 4**, the division times show sensitivity of the cells to their environment. As in the case of the progressive withdrawal of serum, an adaptation is obtained with a recovering effect on the cells after a few passages under the conditions defined.

**Table 4:**

| Condition | 1.2 | 0.5 | 0.3 | plastic |
|---|---|---|---|---|
| d | 1.95 | 1.84 | 1.39 | 1.42 |
| MDT | 12.3 | 13 | 17.3 | 16.9 |

The examples are taken between the passages p154 and p131 for the 3 conditions 1.2 × 10⁶, 0.5 × 10⁶ and 0.3 × 10⁶ feeder cells and between p161 and p139 for the condition on plastic alone.

### Example 6: Deprivation of the cells in growth factors

Under the initial culture conditions, the presence of growth factors is necessary. It is possible to schematically distinguish two families of factors: the cytokines and the trophic factors.

The cytokines are mainly cytokines whose action is through a receptor which is associated with the gp130 protein. Thus, LIF, interleukin 11, interleukin 6, CNTF, oncostatin and cardiotrophin have a similar mode of action with the recruitment at the level of the receptor of a specific chain and the combination of the latter with the gp130 protein in monomeric or sometimes heterodimeric form. In a few cases, the combination of a soluble form of the receptors, a form described inter alia for the receptors for interleukin 6 and CNTF, makes it possible to increase the proliferative effect observed. It has been previously shown that the addition of at least one of these cytokines appeared to be necessary for obtaining embryonic stem cells.

The trophic factors are mainly SCF, IGF-1 and bFGF, which are also used at the start of the culture, as described above. Their presence is also necessary for obtaining and amplifying the cells.

By progressively reducing these growth factors, it is possible to obtain, after a few passages, culture conditions which allow the proliferation of the embryonic or somatic stem cells without the addition of an exogenous growth factor. The different markers used to characterize these cells are always positive for the cells maintained with no factors.

### Example 7: Comparison of the media used

Inoculated into different media, the cells are not obtained with the same frequencies. Comparison of the compositions of the media makes the identification of one of the components in particular difficult. It appears more likely that the whole combination allows an improvement in the physiology of the cells. Among the preferred media, the Ham F12 medium, the MacCoy medium, the DMEM medium and a DMEM medium enriched with biotin will be noted. Starting with such an isolate, adaptation trials are carried out in these different media.

### Example 8: Establishment of the nonadherent cells

During the successive passages of the stem cells, a high-density inoculation directly into the bacteriological dish makes it possible to obtain, after a few passages, embryonic cells which become detached from their substrate and which proliferate in suspension in the form of small regular aggregates. This proliferation is encouraged over several passages by more dilution, mechanical dissociation and nonuse of proteolytic enzyme. The stirring of the cultures is generally carried out but does not represent a distinguishing factor for obtaining non adherent cells. Like the adherent cells, these cells have a characteristic morphology of stem cells, i.e. a small size, a large nucleocytoplasmic ratio, a nucleus with at least one nucleolus which is clearly visible and a very small cytoplasm. These cells are characterized by a growth in small aggregates which are more or less compact. These non adherent cells exhibit cross-reactivity with a number of antibodies, as described above in Pain et al., 1996. These cells are also positive for the endogenous telomerase activity (as presented in example 10 for the EB1, EB4 and EB5 cells). In a non adherent phase, the cells exhibit a high proliferation in different media. The initial inoculation density and the very regular supply of fresh medium provides high densities which may range above 1 × 10⁶ cells per ml. **Table 5** summarizes the main characteristics of a few isolates (parental cells, initial passage of the making into a suspension, number of days maintained in culture in suspension, number of passages and of generations obtained before voluntary stoppage of the maintenances). It can thus be noted that the passage for the making into a suspension can vary from one isolate to another (see isolate EB1 and EB14) and the proliferation rate (see isolate EB3 and EB14).

**Table 5:**

| Name | Parental cells | Initial passage | Start | Days | Passages | Generations |
|---|---|---|---|---|---|---|
| EB1 | S86N16 | p¹¹¹ | 20-01-2001 | 184 | 41 | 120 |
| EB3 | S86N16 | p118 | 23-01-2001 | 381 | 17 | 40 |
| EB4 | S86N45 | p100 | 25-09-2001 | 44 | 17 | 40 |
| EB5 | S86N45 | p100 | 25-09-2001 | 44 | 17 | 40 |
| EB14 | S86N45 | p81 | 05-09-2002 | 70 | 24 | 65 |

It will be noted that the term "start" corresponds to the cells being placed under nonadherence.

We also found that the obtention of non adherent cells is possible after several passages, at any moment, from adherent stem cells that proliferate with or without feeder layer.

### Example 9: Characterization of the established cells

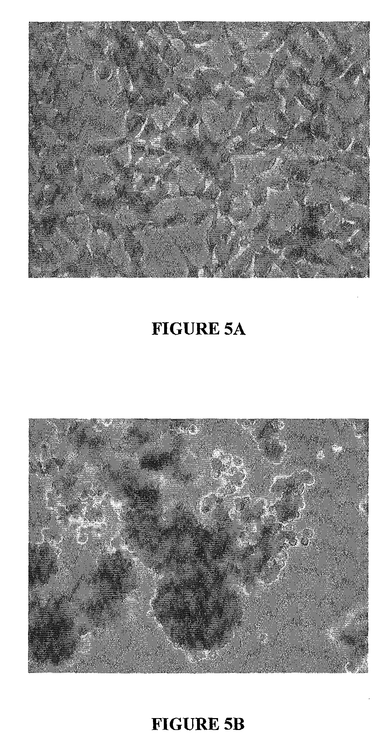

The stem cells maintained for long culture times are characterized with the same criteria as those described above (Pain et al., 1996). It is thus possible to regularly detect the endogenous alkaline phosphatase activity, illustrated by the **photograph of figure 5**, the endogenous telomerase activity and reactivity with specific antibodies such as the antibodies SSEA-1 (TEC-01) and EMA-1.

One of the important criteria during the establishment of the cells is the presence of telomerase. Various tests were carried out during the maintenance of the cells in culture using a TRAP detection kit (Telomerase PCR Elisa, Roche). The cells are detected positive after various passages in culture. Thus, the telomerase activity is detectable for the S86N16 cells, the S86N45 cells and for the EB1, EB4 and EB5 cells which are derived therefrom in a non adherent form (see **table 6**). The CEFs (Chicken Embryonic Fibroblasts) maintained in primary culture are considered as negative. The threshold of an OD < 0.2 is the threshold recommended by the kit as the negative threshold. All the analyses were carried out on an equivalent of 2000 cells.

**Table 6:**

| **Assay of the telomerase activity in various lines at various passages** | | |
|---|---|---|
| Cells | Passage | Telomerase OD |
| S86N16 | p12 | 1.7 |
| | p29 | 2.8 |
| | p185 | 0.97 |
| | p204 | 0.95 |
| S86N16 EB1 | p134 | 1.1 |
| S86N45 | p50 | 0.87 |
| | p58 | 1.1 |
| | p66 | 0.96 |
| | p94 | 1.2 |
| S86N45 EB4 | p112 | 1.4 |
| S86N45 EB5 | p112 | 0.94 |
| CEF* | p4 | 0.07 |

| | | |
|---|---|---|
| * CEF: Chicken Embryonis Fibroblast | | |

### Example 10: Transfection and induction of the cells

The stem cells maintained in a growth over the long term are transfected with various expression plasmids. It has been shown that avian stem cells could be transfected (Pain et al., 1996). In particular, the non adherent cells are transfected and various sorting systems make it possible to identify the stably transfected cells (cell sorting, limiting dilution, and the like). These genetic modifications can be made at the undifferentiated stage of the stem cell. Once this modification has been obtained, the cell is then induced to differentiate spontaneously or by addition of a differentiation inducer. In this case, it is possible to use retinoic acid at concentrations of 10⁻⁸ M to 10⁻⁶ M, or dimethyl sulfoxide at concentrations of 1 to 2% final or sodium butyrate at concentrations of 10⁻⁴ to 10⁻⁸ M, or phorbol ester (TPA, PMA, and the like) or lipopolysaccharides (LPS) at concentrations of 1 to 5 µg/ml final. In another example, the cells can form embryoid bodies in suspension, which embryoid bodies can be caused to adhere to plastic after dissociation or nondissociation of the cells constituting them. These differentiated cells then proliferate but have a more limited capacity for proliferation over the long term. By targeting the genetic modification on a gene which influences the proliferation of the cells, it is possible to make these differentiated cells capable of proliferating over the long term.

### Example 12: Protocol for infecting a non adherent avian cell line (EB1) with a virus

### Amplification of the cells:

The EB1 or EB14 cells are inoculated into a medium, preferably MacCoy's 5A, HAMF12 or DMEM medium, or any other medium of interest, containing 5% serum at a concentration of 0.2 × 10⁶ cells/ml for an initial volume of 50 ml in general. They are maintained in culture at 39°C and at 7.5% CO₂, with stirring. Fresh medium is added every day for the 3 to 4 days for which the amplification lasts in order to reach a cell concentration of 1 to 3 × 10⁶ cells/ml for a final culture volume of 100 to 250 ml.
The cells in suspension are collected and centrifuged for 10 min at 1 000 rpm approximately. The pellet is resuspended in 20 to 50 ml of 1X PBS (Phosphate buffer Salt). The cells are then counted, centrifuged and the pelleted cells are taken up in a serum-free medium at a final concentration of 3 to 5 × 10⁶ cells/ml. Several tubes are then prepared under these conditions containing 3 to 5 × 10⁶ cells per tube.

### Preparation of the virus and infection:

The viral stock having a known titer is rapidly thawed at 37°C and diluted in serum-free medium at a titer of 10 × to 1 000 × the concentration necessary for the final infection. The cells are infected with the virus of interest at an m.o.i. (multiplicity of infection) of 0.01 to 0.5 according to the types of virus, which involves adding between 0.1 and 10% volume/volume of viral suspension to the cellular pellet. After incubating for 1 hour at an optimum temperature for the virus, in general from 33 to 37°C, the cells are again centrifuged and the medium removed with care. This step is found to be often necessary in order to limit the effect of the initial virus in the subsequent process. One of the possibilities is to directly dilute the cells without centrifuging them again with serum-containing medium (5% of serum) at a final concentration of 0.2 to 1 × 10⁶ cells/ml and incubated again.

### Harvesting of the supernatant and of the cells:

After 2 to 4 days of incubation, depending on the viral kinetics and the potential cytopathic effect of certain viruses, the medium containing the cells or the cellular debris is harvested. Depending on the viruses, only the pellet or the supernatant may be of interest and contain the viral particles. The cells are harvested and centrifuged.

The collected supernatant is centrifuged again for 5 to 10 minutes at 2 500 rpm, and stored at -80°C before purification of the particles. An aliquot is collected in order to carry out the titration. The cellular pellet is taken up in 5 ml of serum-free medium, sonicated and centrifuged for 5 to 10 minutes at 2 500 rpm. The supernatant obtained is stored at -80°C up to the purification and the titration of an aliquot.

The viral infection and production efficiencies are compared between the various conditions performed.
For the viruses with cytopathic effects, the titrations are in general carried out by the lysis plaque technique.

### Example 14: Protocol for infecting an adherent avian cell line (S86N45) with a virus

### Preparation of the cells:

The cells are inoculated 48 hours before the infection into T150 flasks at a concentration of between 0.03 and 0.06 × 10⁶ cells/cm² in a medium, preferably MacCoy's 5A, HAMF12 or DMEM medium, or any other medium of interest, containing 5% serum. They are maintained at 39°C and 7.5% CO₂.

### Infection:

The viral stock having a known titer is rapidly thawed at 37°C and diluted in serum-free medium at a titer of 10 × to 1 000 × the concentration necessary for the final infection. The cells are infected with the virus of interest at an m.o.i. (multiplicity of infection) of 0.01 to 0.5 according to the types of virus, which involves adding between 0.1 and 10% volume/volume of viral suspension to the cellular pellet. The infection is generally carried out in a minimum of medium (from 5 to 10 ml for a 75 cm² flask) in a medium containing 0% serum.

After incubating for 1 hour at the optimum temperature for the virus, in general from 33 to 37°C, 20 ml of medium 5% are added to the flasks. In a particular case, the cells can be washed with PBS in order to remove the particles which might be attached to the cells. In the case of a cytopathic virus, the cells are observed daily after the infection in order to monitor the appearance of the cell lysis plaque, which indicates good progress of the infection.

### Harvesting of the supernatant and of the cells:

After 2 to 4 days of incubation, depending on the viral kinetics and the potential cytopathic effect of certain viruses, the medium containing the supernatant, the cells and the cellular debris are harvested. Depending on the viruses, only the pellet or the supernatant may be of interest and contain the viral particles. The cells are harvested and centrifuged. The collected supernatant is centrifuged again for 5 to 10 minutes at 2 500 rpm, and stored at -80°C before purification of the particles. An aliquot is collected in order to carry out the titration. The cellular pellet is taken up in 5 ml of serum-free medium, sonicated and centrifuged for 5 to 10 minutes at 2 500 rpm. The supernatant obtained is stored at -80°C up to the purification and the titration of an aliquot.

The viral infection and production efficiencies are compared between the various conditions performed.
For the viruses with cytopathic effect, the titrations are in general carried out by the lysis plaque technique.

### Example 15: Replication of Modified Vaccinia virus Ankara (MVA) on adherent and nonadherent avian stem cells of the S86N45 line and EB14 line.

The MVA virus (titer 2,5 x 107 TCID50/ml in 0.5 ml vials) was received under frozen conditions.
For safety reasons, the MVA virus and infected cells were kept under controlled conditions (-80°c freezer) and the contaminated plastic material was placed into hypochloride solution for more than 1 hour and then place into a bag for full and complete autoclave inactivation.

### Experiments and results

The S86N45 and EB14 cells were thawed in a HamF12 based complete medium. The adherent S86N45 cells were amplified quite rapidly, with a good growth rate and a nice morphology..
The cells were infected 1 hour in 2 ml of PBS with the different m.o.i. of interest with no washing with PBS after the infection. The medium was just added to the complete infectious medium, i.e. the added virus was not removed.

After 3 days of infection, the cell lysis appears to be proportional to the used m.o.i. This cytopathic effect is a good indicator of the virus infection of the cells. So cells and supernatant are harvested and stored at - 80°C before purification of particles and/or titration.

The non adherent EB14 cells were amplified. The cells were infected, not washed, and the complete medium directly added on the inocculum after 1 hour of contact with viral particles. After 3 days, a characteristic cell lysis was observed. The non infected cells used as the control were counted and a good growth was demonstrated, showing good culture conditions and therefore confirming an efficient lysis by the virus in the infected culture. Cells and supernatant are harvested and stored at - 80°C before purification of particles and/or titration (see table 7).

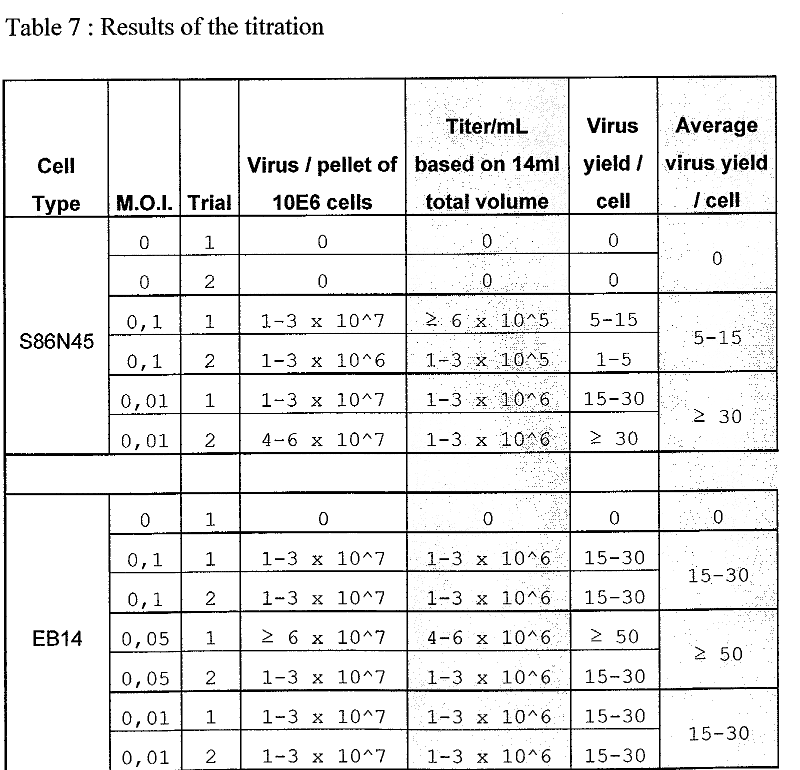

### REFERENCES

Baba TW, Humphries EIL (1985). Formation of a transformed follicle is necessary but not sufficient for development of an avian leukosis virus-induced lymphoma. Proc. Nail. Acad. Sci. USA 82: 213-216.

Beug H, von Kirchbach A, Doderlein G, Conscience JF, Graf T. (1979). Chicken hematopoietic cells transformed by seven strains of defective avian leukemia viruses display three distinct phenotypes of differentiation. Cell 18: 375-390.

Guilhot C, Benchaibi M, Flechon JE, Samarut J. (1993). The 12S adenoviral E1A protein immortalizes avian cells and interacts with the avian RB product. Oncogene 8:619-624

Kawaguchi T, Nomura K, Hirayama Y, Kitagawa T. (1987). Establishment and characterization of a chicken hepatocellular carcinoma cell line, LMH. Cancer Res 1987 47: 4460-4464.

Kim H, You S, Farris J. Foster LK, Foster DN. (2001). Post-transcriptional inactivation of p53 in immortalized chicken embryo fibroblast cells. Oncogene 20: 3306-3310.

Kim H, You S, Kim IJ, Foster LK, Farris J, Ambady S, Ponce de Leon FA, Foster DN. (2001). Alterations in p53 and E2F-1 function common to immortalized chicken embryo fibroblasts. Oncogene 20: 2671-2682.

Liu JL, Klein PA, Moscovici MG, Moscovici C. (1992). Monoclonal antibodies recognizing normal and retrovirus-transformed chicken hematopoietic cells. Virology 189: 583-591.

Moscovici C, Moscovici MG, Jimenez H, Lai MM, Hayman MJ, Vogt PK. (1977). Continuous tissue culture cell lines derived from chemically induced tumors of Japanese quail. Cell 11: 95-103.

Moss B. (1994) Replicating and host-restricted non-replicating vaccinia virus vectors for vaccine development. Dev Biol Stand. 82: 55-63.

Pain B., Clark M.E., Shen M., Nakazawa H., Sakurai M., Samarut J., Etches RJ. (1996). Long-term in vitro culture and characterisation of avian embryonic stem cells with multiple morphogenetic potentialities. Development 122: 2339-2348.

Pain B., Chenevier P., Samarut J. (1999). Chicken embryonic stem cells and transgenic strategies. Cells Tissues Organs 165: 212-219.

Samarut J, Gazzolo L. (1982). Target cells infected by avian erythroblastosis virus differentiate and become transformed. Cell 28: 921-929.

Smith JR and Pereira-Smith OM (1996). Replicative senescence: implications for in vivo aging and tumor suppression. Science 273, 63-67.

## Claims

1. A method for replicating orthopoxviruses and recombinants derivatives such as native or recombinant vaccinia virus comprising the steps of inoculating avian stem cells with viral particles and culturing said cells in a basal medium until cells lysis occurs and newly produced viral particles are released in said medium.

2. A method according to claim 1, wherein inoculation is performed with an m.o.i. (multiplicity of infection) of 0.01 to 0.5.

3. A method according to one of claims 1 to 2, wherein said vaccinia virus is the modified vaccinia virus such as Modified Vaccinia virus Ankara (MVA) or a recombinant vaccinia virus.

4. A method according to one of claims 1 to 3 for producing a vaccine against smallpox.

5. A method according to one of claims 1 to 4, wherein said avian stem cell lines are obtainable by a process consisting of:
a) culturing avian cells in a medium containing all the factors allowing their growth and an inactivated feeder layer,
b) passage by modifying the culture medium so as to obtain progressive or total withdrawal of said factors, of the serum and/or of the feeder layer,
c) establishing adherent or non adherent cell lines capable of proliferating in a basal medium in the absence of exogenous growth factors, serum and/or inactivated feeder layer.

6. A method according to claim 5, wherein said avian stem cells obtained in step c) are capable of proliferating for at least 600 days.

7. A method according to one of claims 5 to 6, wherein said avian stem cells are avian embryonic stem cells or avian somatic stem cells.

8. A method according to one of claims 5 to 7, wherein step b) consists in a withdrawal of the components of the medium (growth factors alone or serum alone or growth factors and then serum or alternatively serum and then growth factors).

9. A method according to one of claims 5 to 7, wherein step b) consists in a progressive or total withdrawal of the feeder layer and then optionally in a withdrawal of the other components of the medium (growth factors and serum).

10. A method according to one of claims 5 to 9, wherein said avian cell lines are nonadherent stem cells which proliferate in suspension in a medium free of exogenous growth factors.

11. A method according to one of claims 5 to 9, wherein said avian cell lines are nonadherent stem cells which proliferate in suspension in a medium free of serum (serum-free medium).

12. A method according to one of claims 1 to 9, wherein said avian cell lines are nonadherent stem cells which proliferate in suspension in a medium free of exogenous growth factors and serum.

13. A method according to one of claims 1 to 13, wherein said avian cell lines have at least one of the following characteristics:
- a high nucleocytoplasmic ratio,
- an endogenous alkaline phosphatase activity,
- an endogenous telomerase activity,
- a reactivity with specific antibodies selected from the group of antibodies SSEA-1 (TEC01), SSEA-3, and EMA-1.

14. A method according to one of claims 1 to 13, wherein said avian cell lines are cultivated in basal medium, in particular in a medium such as DMEM, GMEM, HamF12 or McCoy supplemented with additives such as nonessential amino acids, vitamins and sodium pyruvate.

15. A method to produce live or attenuated vaccine such as a vaccine against smallpox comprising culturing the adherent or non adherent cell lines established in step c) according to the process defined in one of claims 5 to 14, inoculating said cells with viral particles and culturing said cells in a basal medium as mentioned above until cell lysis occurs and newly produced viral particles are released in said medium.

16. The use of the non-adherent cells as defined in one of claims 10 to 12 to produce live or attenuated vaccine belonging to the family of orthopoxvirus, in particular vaccinia virus, modified vaccinia virus such as Modified Vaccinia virus Ankara (MVA) and recombinant vaccinia virus.

17. The use according to claim 16 for producing a vaccine against smallpox.

18. The use according to claim 16 for producing a vaccine against cancer.
